# EUROPEAN PATENT SPECIFICATION

(11) **EP 1 885 373 B1**
(45) Date of publication and mention of the grant of the patent: **20.05.2015**
(21) Application number: 06750845.7
(22) Date of filing: 20.04.2006
(51) Int. Cl.: A61K 31/56, A61L 9/04, A61K 31/045, A61K 31/573, A61K 47/14, A61P 17/06

(54) **USE OF A CLOBETASOL SPRAY FORMULATION TO TREAT PSORIASIS**
VERWENDUNG EINER CLOBETASOL-SPRAYFORMULIERUNG ZUR BEHANDLUNG VON PSORIASIS
UTILISATION D'UNE FORMULATION PULVERISABLE AU CLOBETASOL POUR TRAITER LE PSORIASIS

(30) Priority: 25.04.2005 US 674946 P
(43) Date of publication of application: 13.02.2008
(73) Proprietor: Dow Pharmaceutical Sciences, Petaluma, CA 94954-6542 (US); Dermalogix Partners, Inc., Scarborough, ME 04070 (US)
(72) Inventor: DOW, Gordon, J., Santa Rosa, California 95409 (US); STEWART, Daniel, M., Bloomfield Hills, Michigan 48304 (US)
(74) Representative: Gowshall, Jonathan Vallance
(86) International application number: PCT/US2006/014903
(87) International publication number: WO 2006/115987

(56) References cited:
- US-A- 5 972 920
- US-A1- 2003 007 929
- CRUTCHFIELD CHARLES E III: "The temporal dermatohistopathologic examination of human psoriatic skin treated with a novel topical liquid formulation containing clobetasol propionate" WORLD WIDE WEB JOURNAL, SEBASTOPOL, CA, US, US, vol. 3, no. 4, 16 June 1998 (1998-06-16), XP009135802 ISSN: 1085-2301
- HOUSEMAN T.S. ET AL.: 'The use of 0.25% zinc pyrithione spray does not enhance the efficacy of clobetasol propionate 0.05% foam in the treatment of psoriasis' JOURNAL OF THE AMERICAN ACADEMY OF DERMATOLOGY vol. 49, no. 1, 2003, pages 79 - 82, XP003004498

## Description

### Field of the Invention

The present invention relates to a composition for use in a method for treating psoriasis with clobetasol.

### Background of the Invention

Psoriasis is a lifelong disorder with onset at anytime throughout life, affecting about 2 to 3% of the US population. It affects men and women equally and afflicts almost all races in varying frequency rates. Psoriasis usually appears first between the ages of 15 and 30 years and may occur anywhere on the body. Psoriasis causes significant psychological and social distress, and significantly impacts quality of life.¹ Unsatisfactory treatment of the disorder has a considerable adverse impact on patient's quality of life with patients complaining about the messiness of the topical agents used.²⁻⁶

Topical clobetasol propionate is a corticosteroid that is currently one of the most used treatments for psoriasis and its safety and efficacy is well defined in the medical literature.⁷ However, current cream and ointment formulations of clobetasol present disadvantages such as being greasy and difficult to apply on large areas, which disadvantages negatively impact treatment compliance and quality of life. Additionally, certain patient populations suffering from psoriasis in difficult to reach areas, including singles, elderly patients, or patients with physical handicaps may have difficulty applying the conventional formulations on the lesions.

Further, with regard to the use of conventional cream and ointment formulations, long-term continuous topical therapy should be avoided where possible, particularly in infants and children, as adrenal suppression can occur readily even without occlusion of the applied medication on the skin.

If used in childhood or on the face, courses using conventional cream and ointment formulations often are limited if possible to five days and occlusion should not be used.

The face, more than other areas of the body, may exhibit atrophic changes after prolonged treatment with potent topical corticosteroids. This must be borne in mind when treating such conditions as psoriasis, discoid lupus erythematosus and severe eczema.

Topical corticosteroids in conventional formulations may be hazardous in psoriasis for a number of reasons including rebound relapses, development of tolerance, risk of generalized pustular psoriasis and development of local or systemic toxicity due to impaired barrier function of the skin. Thus, if used in psoriasis, careful patient supervision is important.

Prolonged use of large amounts of topical corticosteroids, or treatment of extensive areas, can result in sufficient systemic absorption to produce the features of hypercorticism.

Provided the weekly dosage is less than 50 g in adults, any suppression of the HPA (Hypothalamic-Pituitary-Adrenal) axis is likely to be transient with a rapid return to normal values once the short course of steroid therapy has ceased. The same applies to children given proportionate dosage. Use of occlusive dressing increases the absorption of topical corticosteroids. In infants a napkin may act as an occlusive dressing.

Prolonged and intensive treatment with a highly active corticosteroid in conventional preparations may cause local atrophic changes in the skin such as thinning (atrophy), striae and dilatation of the superficial blood vessels (telangiectasia), particularly when occlusive dressings are used or when skin folds are involved.

In rare instances, treatment of psoriasis with corticosteroids (or its withdrawal) is thought to have provoked the pustular form of the disease.

There are also reports of pigmentation changes and hypertrichosis with topical steroids.

Gottlieb et al, J. Cutaneous Med. Surg., 7(3):185-192 (2003), disclose a clobetasol propionate foam composition that is as effective in treating scalp psoriasis as is a clobetasol propionate solution and was judged to be superior in a two-week treatment of non-scalp psoriasis than a comparable ointment, gel, or cream. The treatment in Gottlieb was limited to a period of two weeks due to the potential for systemic and topical adverse effects.

Crutchfield C., World Wide Web Journal, Vol 3, No 4, 1998, discloses use of clobetasol propionate to treat psoriasis.

### SUMMARY OF THE INVENTION

The invention is defined in the claims.

In an effort to expand upon the current treatment options for patient groups experiencing difficulties adequately treating their psoriasis (singles, patients with physical handicap, elderly patients) or patients seeking to minimize the time spent on their treatment, a new spray formulation of this potent corticosteroid was developed.

Accordingly, one embodiment of the present invention provides an easy to apply spray formulation of clobetasol propionate 0.05% to solve the compliance issues without compromising the required efficacy or resulting in significant adverse effects. The spray formulation of the invention in a preferred embodiment is not a foam. Rather, it is a clear solution that is applied to the skin as a transparent or substantially transparent liquid that is left on the skin or gently rubbed into the skin.

In contrast to the expected adverse effects with prolonged treatment with clobetasol propionate in the conventional formulations, the treatment regime with the spray formulation of the present invention for a period of 4 weeks increased clinical benefit with no detectable adverse events except for mild or moderate burning sensation. The increased clinical benefits include additional clearing and improvement of the psoriasis. No cases of telangiectasia, skin atrophy or HPA axis suppression were detected.

It has been unexpectedly discovered that treatment of psoriasis lesions with the spray formulation of the invention provides superior therapeutic results than those obtained with a prior art topical foam formulation as reported in Gottlieb et al (Reference 10). Therapeutic results after two weeks of treatment or after four weeks of treatment in accordance with the present invention provides superior relief from symptoms of psoriasis than that which is obtainable with prior art formulations, including the foam formulation as reported in Gottlieb et al.

The present invention therefore provides a composition for use in a method for treating psoriasis, by spraying onto the skin with psoriasis daily, preferably at least twice daily, for at least 4 weeks a composition containing 0.005% to 1% by weight of clobetasol propionate. The composition that is sprayed onto the skin is a non-foaming solution of clobetasol propionate, which provides effective relief from symptoms of psoriasis without the messiness of gels, ointments, or foams.

The composition contains 0.005% to 1% by weight of clobetasol propionate, more preferably 0.05% by weight of clobetasol propionate. Further, the composition additionally contains an anionic surfactant, and a carrier. If desired, the composition may contain additional active compounds. One such example is an antimicrobial agent such as undecyclenic acid. The anionic surfactant is preferably sodium lauryl sulfate.

The carrier is a mixture that contains a solvent compound useful for spray formulations and an emollient compound. Nonlimiting examples of suitable solvent compounds are volatile compounds such as alcohol (ethyl alcohol), isopropyl alcohol, cyclomethicone and acetone. Emollient compounds suitable for the carrier include non-volatile compounds such as various esters of monohydric alcohols and fatty acids with a chain length from 8 to 22 and triglycerides that are liquid at room temperature. The preferred solvent compound is alcohol and the preferred emollient compounds are isopropyl myristate and isopropyl palmitate, with isopropyl myristate being the most preferred. On a weight basis, the ratio of solvent compound to emollient compound in the carrier for the spray is from 5:1 to 1:5. Preferably the ratio is 3:1 to 1:3, and most preferably the ratio is 1.5:1 to 1:1.5.

A preferred composition of the present invention contains clobetasol propionate, alcohol, isopropyl myristate, anionic surfactant such as sodium lauryl sulfate, and optionally an antimicrobial compound such as an antifungal compound like undecylenic acid. More preferably, the composition of the present invention contains 0.05% by weight of clobetasol propionate, 49.25% by weight of 92.8% ethanol, 50.30% by weight of isopropyl myristate, 0.1% by weight of sodium lauryl sulfate, and 0.3% by weight of undecylenic acid.

Unlike prior art compositions, such as disclosed in US Patent No. 5,972,920, which contain zinc pyrithione as an active anti-psoriatic agent, the compositions of the invention, in a preferred embodiment, are free of, or substantially free of zinc pyrithione and preferably are substantially free of zinc. It has been unexpectedly discovered that, even without zinc pyrithione, the composition of the invention maintains its effectiveness in treating psoriasis and is well tolerated.

The preferred method for treating psoriasis is by spraying onto the skin afflicted with psoriasis a composition containing 0.05% by weight of clobetasol propionate, 49.25% by weight of 92.8% ethanol, 50.30% by weight of isopropyl myristate, 0.1% by weight of sodium lauryl sulfate, and, if desired, 0.3% by weight of undecylenic acid.

The composition used in the method of treating psoriasis may be packaged in a bottle fitted with a spray pump closure that can be mechanically actuated by a patient to apply the compostion to the affected skin (pump type spray). An alternative spray embodiment of this invention is an aerosol type spray of the composition in which an aerosol can or bottle with an actuator is charged with a propellant. A nonlimiting preferred aerosol embodiment may be prepared with 95% of the composition and 5% of the propellent. A preferred propellant mixture is 85% isobutene and 15% propane.

Other objects and features of the present invention will become apparent from the following detailed description considered in conjunction with the accompanying drawings. It is to be understood, however, that the drawings are designed solely for purposes of illustration and not as a definition of the limits of the invention, for which reference should be made to the appended claims. It should be further understood that the drawings are not necessarily drawn to scale and that, unless otherwise indicated, they are merely intended to conceptually illustrate the structures and procedures described herein.

### BRIEF DESCRIPTION OF THE DRAWINGS

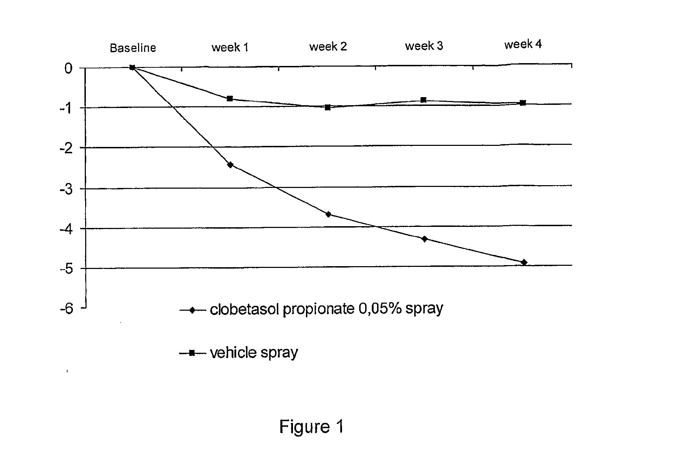

Figure 1 is a graph of the change from Baseline in Overall Target Plaque Psoriasis Score comparing a spray of the invention to a spray containing its vehicle alone.

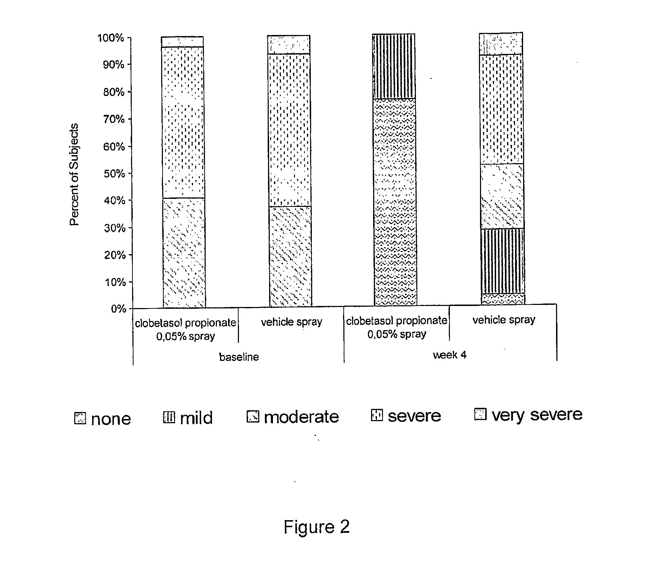

Figure 2 is a bar graph comparing Overall Target Plaque Psoriasis Assessment at Baseline and at 4 weeks using either a spray of the invention or a spray containing its vehicle alone. At week 4 the difference in percent of subjects with none or mild psoriasis was significantly in favor of clobetasol propionate spray (p<0.001). There was no statistically significant difference between the target lesions at baseline.

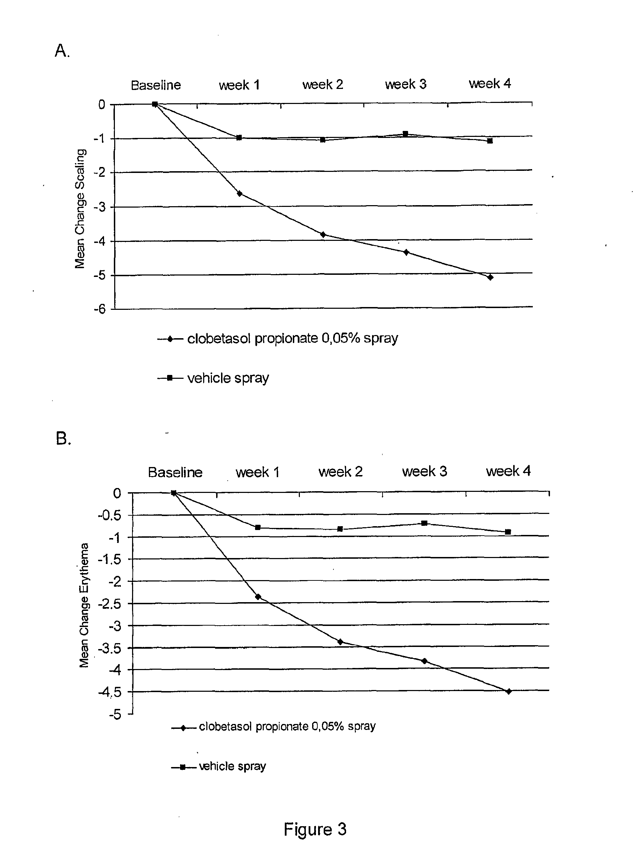

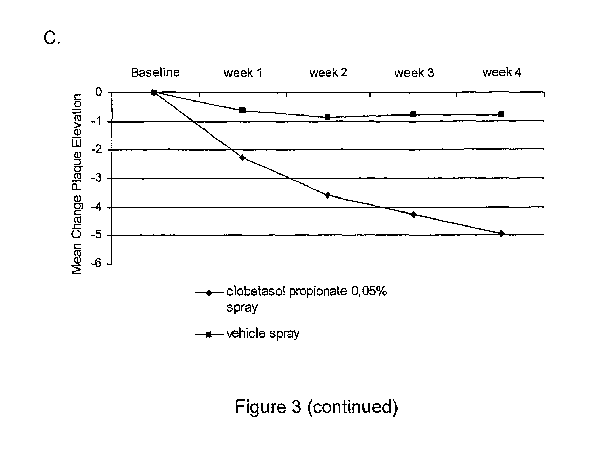

Figure 3 is a series of graphs comparing (a) scaling, (b) erythema, and (c) plaque elevation following treatment with a spray of the invention compared to treatment with a spray containing its vehicle alone. The results show a decrease from baseline for signs of psoriasis of (a) scaling, (b) erythema, and (c) plaque elevation.

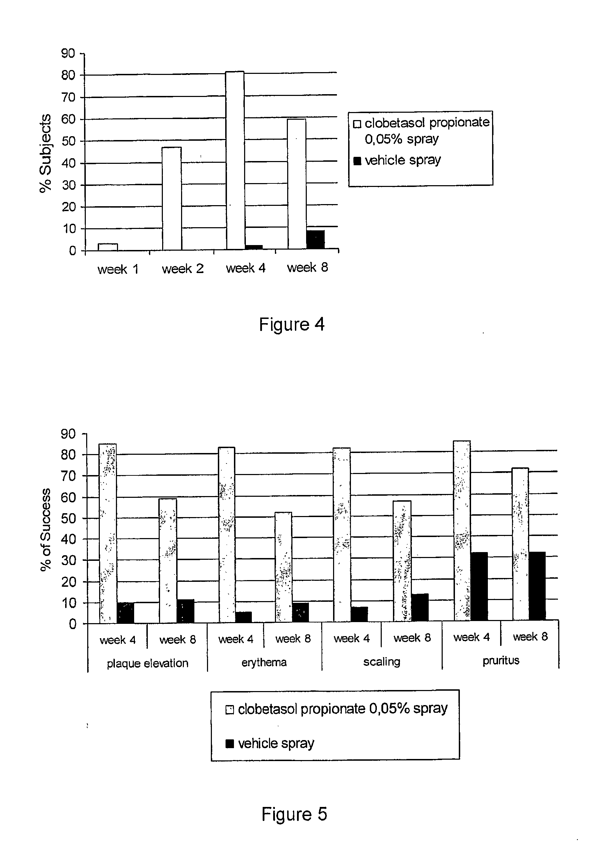

Figure 4 is a bar graph comparing percentage of subjects cleared or almost cleared following treatment with either a spray of the invention or a spray containing vehicle alone.
Figure 5 is a bar graph comparing the percentage success rate in relieving signs and symptoms of psoriasis when using either a spray of the invention or its vehicle spray for 4 weeks followed by 4 weeks of no treatment. Treatment with clobetasol propionate 0.05% spray resulted in a significantly higher clinical success rate (p < 0.001) compared with the vehicle spray.

### DETAILED DESCRIPTION OF A PREFERRED EMBODIMENT OF THE INVENTION

Two studies were performed to evaluate the efficacy and safety of clobetasol propionate 0.05% in the present spray formulation in the treatment of plaque psoriasis.

### A. First Study

### METHODS

### Study design

- Four week single center, randomized, double-blind, vehicle-controlled, intra-individual, pilot study.

### Subject selection

- Male or female subjects, at least 18 years of age with two bilaterally distributed psoriasis plaques of equivalent size, each between 5 cm² and 100 cm².
- The subjects have overall target plaque severity score greater than or equal to 5 on a scale of 0 (no evidence of disease) to 8 (very severe overall plaque elevation, scaling, and/or erythema of the target plaque).

### Treatments

- Target areas were randomized in a 1:1 ratio to receive either clobetasol propionate spray, or its vehicle spray ;
- Medication was applied twice-daily for 4 weeks to the target lesions.
-

### Efficacy and safety assessments

- Overall target plaque severity score was obtained at all visits ;
- Treatment success rated at week 4 and defined as percentage of subjects with an overall target plaque severity score of 0 or 1 ;
- Scaling, erythema, and plaque elevation scores on a scale from 0 (none) to 4 (severe/very severe) were obtained at all visits ;
- Adverse events were reported throughout the study.

### RESULTS

### Subjects studied

- Twenty-seven subjects with plaque psoriasis entered the study. Demographic data are provided in Table 1 ;
- A total of 25 subjects completed the study: 2 subjects withdrew for administrative reasons.

### Efficacy

Results for the mean score decrease at week 4 of overall target plaque severity was statistically significant (p<0.001) in favor of clobetasol propionate 0.05% spray (-4.96 change in severity score) relative to its vehicle (-0.96 change in severity score); (Figure 1).

After 4 weeks of treatment, success was statistically significant (p<0.001) in favor of clobetasol propionate 0.05% spray (100%) relative to the vehicle (28%); (Figure 2).

Additionally, mean score decrease at all visits from baseline for signs and symptoms was significantly in favor (p<0.001) of clobetasol propionate 0.05% spray from week 1 on (Figure 3).

There was a significant within subject treatment effect (p<0.001) with an average difference in scores of 4.08 in favor of the clobetasol propionate 0.05% spray versus its vehicle.

### Safety

From the 21 adverse events reported by 14 subjects, only one local adverse event (mild burning of the target lesion) was considered probably related to study medication.

No case of skin atrophy or telangiectasia was reported.

No serious adverse events occurred during the course of the study.

### CONCLUSION

Clobetasol propionate 0.05% spray was effective in reducing the severity of target plaque psoriasis, scaling, erythema, and plaque elevation as early as week 1 compared to vehicle. Further, clobetasol propionate 0.05% spray was well tolerated by the subjects who participated in this clinical study.

**Table 1. Subject demographics**

| **Number** | 27 |
|---|---|
| **Age** | |
| Mean ± SD | 51.59 ± 12.76 |
| Range | 21.0-75.0 |
| **Gender** | |
| Male | 18 (67%) |
| Female | 9 (33%) |
| **Race** | |
| White | 23 (85%) |
| Black | 1 (4%) |
| Hispanic/Latino | 2 (7%) |
| Other | 1 (4%) |

### B. Second study

### OBJECTIVE

To compare the efficacy and safety of clobetasol propionate spray to its vehicle in larger patient pool with moderate to severe plaque-type psoriasis. The spray formulation contains clobetasol propionate: 0.05% w/w, alcohol (92.8% w/w ethanol): 49.25% w/w, isopropyl myristate: 50.30% (w/w), sodium lauryl sulfate: 0.1% (w/w), and undecylenic acid: 0.3%.

### METHODS

### Study design

Four week, multicenter, randomized, double-blind, vehicle-controlled, parallel-group, comparative study.

### Subject selection

Male or female subjects, at least 18 years of age, presenting with an area of plaque psoriasis of at least 2% of the body surface area (excluding face, scalp, groin, axillae and other intertriginous areas).

The subjects exhibited overall target plaque severity score (sum of plaque elevation, scaling, and erythema) of at least 3 on a scale ranging from 0 = none to 4 = severe/very severe.

### Treatments

Suitable subjects were randomized in a 1:1 ratio to receive either clobetasol propionate 0.05% spray or its vehicle spray.

Medication was to be applied twice-daily for 4 weeks to the target lesions ;

Treatment phase was followed by a 4-week non-treatment follow-up.

### Efficacy and safety assessments

- Success rate of the overall disease severity score (scores for all psoriasis signs). Success was defined as a grade of 2 or less on a 0 to 4 scale (0 = clear to 4 = severe/very severe) at week 1 and 2 and as a grade of 1 or less at week 4 and 8;
- Psoriasis signs (scaling, erythema, plaque elevation) and pruritus were scored on a scale from 0 to 4 at all visits.
- Treatment success rates of signs and symptoms, defined as a grade of 1 or less ;
- Adverse events reported throughout the study. Expected local adverse events included skin atrophy, telangiectasia, burning/stinging, and folliculitis.
- Clinical evaluation of HPA axis suppression was observed at every study visit. Subjects were directly queried about clinical signs and symptoms of adrenal suppression.

Study evaluations took place at week 1, 2, 4, and 8.

### RESULTS

### Subjects studied

- A total of 120 subjects completed the study
- Demographic data are provided in Table 2;

### Efficacy assessments

At the end of treatment
∘ A total of 81% of subjects in the clobetasol propionate 0.05% spray group were considered treatment success (score of 0 or 1 on 4 point scale) compared to 2% of subjects in the vehicle treatment group. This difference was statistically significant (p < 0.001, Figure 4). Subjects that were clear or almost clear with clobetasol propionate 0.05% spray increased by 75% from 28 of 60 subjects at Week 2 to 49 of 60 subjects at week 4 of treatment. The additional two weeks of therapy from week 2 to week 4 resulted in 18 subjects with complete clearing of their disease compared to no clear subjects in that group at Week 2 ;
∘ Success rates (Figure 5) for scaling (82%), erythema (83%), plaque elevation (85%), and pruritus (85%) were significantly in favor of clobetasol propionate 0.05% spray compared to the vehicle spray (7%, 17%, 10% and 32%, respectively; p < 0.001) ;

At the end of 4 weeks of treatment free follow-up
∘ The evaluation of the overall disease severity demonstrated a statistically significant difference (p < 0.001) for subjects considered treatment success between the 59% of subjects (34 of 58) in the clobetasol propionate 0.05% spray group and the 8% of subjects treated with the vehicle spray (Figure 4);
∘ Success rates of scaling (57%), erythema (52%), plaque elevation (59%), and pruritus (72%) were significantly in favor of clobetasol propionate 0.05% spray (p < 0.001; Figure 5).

### Safety evaluations

- There were no serious adverse events reported during the study ;
- Incidence rates of adverse events were similar in both groups ;
- The most frequent treatment-related adverse event reported in each treatment group was burning, mostly reported as mild to moderate in severity ;
- There were no cases of telangiectasia or skin atrophy reported with clobetasol propionate spray ;
- There was no clinically detectable HPA axis suppression.

### CONCLUSION

- The increase of the treatment period from 2 to 4 weeks increased clinical benefit with additional clearing and improvement of disease with no substantial change in the safety profile.
- Clobetasol propionate 0.05% spray applied for 4 weeks showed a better efficacy profile than other available clobetasol propionate formulations applied over the same treatment duration^{8,9};
- The success rate of clobetasol propionate 0.05% spray at 8 weeks confirms a durable clinical response and unexpectedly there was no rebound effect;
- Clobetasol propionate 0.05% spray is safe and well tolerated;

**Table 2. Subject Demographics**

| | **Clobetasol Propionate 0.05% Spray** | **Vehicle Spray** |
|---|---|---|
| **Number of Subjects Age (Years)** | 60 | 60 |
| Mean±SD | 46.17±13.26 | 45.90±13.37 |
| Range | 18.0-81.0 | 18.0-77.0 |
| **Gender** | | |
| Male | 31 (52%) | 37 (62%.) |
| Female | 29 (48%) | 23 (38%) |
| **Race** | | |
| White | 50 (83%) | 53 (88%) |
| Non-White | 10 (17%) | 7 (12%) |
| Black | 3 (5%) | 1 (2%) |
| Asian/Pacific Islander | 2 (3%) | 1 (2%) |
| Hispanic/Latino | 4 (7%) | 4 (7%) |
| American/Alaskan Native | 1 (2%) | 0 (0%) |
| Other | 0 (0%) | 1 (2%) |

The present invention provides several unexpected advantages over prior art compositions containing clobetasol and the use of such prior art compositions to treat topical diseases such as psoriasis. The present composition of the invention provides an effective therapy for such topical, diseases that is more rapidly obtained than with prior art methods and compositions containing clobetasol propionate. The present method and composition also provide a higher degree of effectiveness than is obtained with prior art methods and compositions containing clobetasol propionate. The present method and composition also provides an increased durability of treatment with little or no rebound effect. Thus, when utilizing the present invention, there is less reversion to a diseased state upon discontinuation of treatment than occurs with presently known methods and compositions of clobetasol propionate. There is also little or no flare-up of disease following discontinuation of therapy with the present invention compared to what occurs following discontinuation of therapy with presently known methods and compositions of clobetasol propionate.

### • REFERENCES

1. Rapp SR, Exum ML, Reboussin DM, et al. The physical, psychological and social impact of psoriasis. J Health Psychol 1997;2: 525-537.
2. Koo J. Population-based epidemiologic study of psoriasis with emphasis on quality of life assessment. Dermtol Clin. 1996; 14:485-496.
3. Rapp SR, Feldman SR, Exum ML, Fleischer AB Jr Reboussin DM. Psoriasis causes much disability as other major medical diseases. J Am Acad Dermatol. 1999;41(3 Pt 1):401-410.
4. Lebwohl M, Sherer D, Washenik K, Krueger GG, Menter A, Koo J, Feldman SR. A randomized, double-blind, placebo-controlled study of clobetasol propionate 0.05% foam in the treatment of nonscalp psoriasis. Int J Dermatol. 2002;41(5):269-274.
5. Rapp SR, Feldman SR, Fleischer Jr AB, Reboussin DM, Exum ML. Health related quality of life in psoriasis: A biopsychosocial model and measures. In Rajagopalan R, Sheretz EF, Anderson R, eds. Care Management of Skin Diseases: Life Quality and economic Impact. New York: Marcel Dekker, Inc, 1998;125-145.
6. Arruda LH, De Moraes AP. The impact of psoriasis on quality of life. Br J Dermatol. 2001;144 Suppl 58:33-36.
7. Amin S., Cornell R., Soughton R., Maibach H. In Psoriasis, Third Edition Ed. Marcel Dekker, 1998;453-467.
8. Jorizzo JL, Magee K, Stewart DM, et al. Clobetasol propionate emollient 0.05%: Hypothalamic-pituitary-adrenal-axis safety and four -week clinical efficacy results in plaque-type psoriasis. Cutis. 1997; 60: 55-60.
9. Goldberg B, Hartdegen R, Presbury D, Smith EH, et al. A double blind, multicentre comparison of 0.05% halobetasol propionate ointment and 0.05% clobetasol propionate ointment in patients with chronic, localized plaque psoriasis. J Am Acad Dermatol. 1991; 25: 1145-1148.
10. Gottlieb AB, Ford RO, Spellman MC, The efficacy and tolerability of Clobetasol Propionate Foam 0.05% in the treatment of mild to moderate plaque-type psoriasis of nonscalp regions. J. Cutaneous Med and Surg. 2003; 7(3):185-192.

Thus, while there have shown and described and pointed out fundamental novel features of the invention as applied to a preferred embodiment thereof, it will be understood that various omissions and substitutions and changes in the form and details of the devices illustrated, and in their operation, may be made by those skilled in the art without departing from the claimed invention. For example, it is expressly intended that all combinations of those elements and/or method steps which perform substantially the same function in substantially the same way to achieve the same results are within the scope of the claimed invention. Moreover, it should be recognized that structures and/or elements and/or method steps shown and/or described in connection with any disclosed form or embodiment of the invention may be incorporated in any other disclosed or described or suggested form or embodiment as a general matter of design choice. It is the intention, therefore, to be limited only as indicated by the scope of the claims appended hereto.

## Claims

1. A composition comprising clobetasol propionate, a solvent compound and an emollient wherein the composition comprises 0.05% by weight of clobetasol propionate, for use in a method for treating an individual suffering from psoriasis lesions affecting at least 2% of the surface area of the body of the individual by spraying the composition onto the psoriasis lesions daily for at least 4 weeks.

2. The composition for use in a method of claim 1 wherein the composition is free of zinc pyrithione.

3. The composition for use in a method of claim 1 or 2 wherein the composition is sprayed twice daily for at least four weeks.

4. The composition for use in a method of any preceding claim wherein the solvent compound is ethyl alcohol.

5. The composition for use in a method of any preceding claim wherein the emollient is isopropyl myristate.

6. The composition for use in a method of any preceding claim wherein the composition comprises alcohol, isopropyl myristate, and sodium lauryl sulfate.

7. The composition for use in a method of any preceding claim wherein the carrier comprises a solvent compound and an emollient compound in a ratio on a weight basis of between 5:1 and 1:5,

8. The composition for use in a method of claim 7 wherein the ratio is preferably between 3:1 and 1:3.

9. The composition for use in a method of any preceding claim wherein the composition, following the spraying onto the skin, is left on the skin or is rubbed into the skin.

10. The composition for use in a method of any preceding claim wherein the composition is a non-foaming solution.

11. The composition for use in a method of any preceding claim wherein the composition comprises an anionic surfactant.

12. The composition for use in a method of any preceding claim which comprises 0.05% by weight of clobetasol propionate, 49.25% by weight of 92.8% ethanol, 50.30% by weight of isopropyl myristate, and 0.1% by weight of sodium lauryl sulfate.

13. The composition for use in a method of any preceding claim wherein the method does not cause significant clinically detectable HPA axis suppression.

## Patentansprüche

1. Zusammensetzung umfassend Clobetasolpropionat, eine Lösungsmittelverbindung und ein Emolliens, worin die Zusammensetzung 0,05 Gewichts-% Clobetasolpropionat umfasst, zur Verwendung in einem Verfahren zur Behandlung eines Individuums, das an Psoriasis-Läsionen leidet, die zumindest 2 % der Körperoberfläche des Individuums betreffen, indem die Zusammensetzung zumindest 4 Wochen lang täglich auf die Psoriasis-Läsionen gesprüht wird.

2. Zusammensetzung zur Verwendung in einem Verfahren nach Anspruch 1, worin die Zusammensetzung von Zinkpyrithion frei ist.

3. Zusammensetzung zur Verwendung in einem Verfahren nach Anspruch 1 oder 2, worin die Zusammensetzung zumindest 4 Wochen lang zweimal täglich aufgesprüht wird.

4. Zusammensetzung zur Verwendung in einem Verfahren nach irgendeinem vorhergehenden Anspruch, worin die Lösungsmittelverbindung Ethylalkohol ist.

5. Zusammensetzung zur Verwendung in einem Verfahren nach irgendeinem vorhergehenden Anspruch, worin das Emolliens Isopropylmyristat ist.

6. Zusammensetzung zur Verwendung in einem Verfahren nach irgendeinem vorhergehenden Anspruch, worin die Zusammensetzung Alkohol, Isopropylmyristat und Natriumlaurylsulfat umfasst.

7. Zusammensetzung zur Verwendung in einem Verfahren nach irgendeinem vorhergehenden Anspruch, worin der Träger eine Lösungsmittelverbindung und eine Emolliensverbindung in einem Verhältnis auf einer Gewichtsbasis von zwischen 5:1 und 1:5 umfasst.

8. Zusammensetzung zur Verwendung in einem Verfahren nach Anspruch 7, worin das Verhältnis vorzugsweise zwischen 3:1 und 1:3 ist.

9. Zusammensetzung zur Verwendung in einem Verfahren nach irgendeinem vorhergehenden Anspruch, worin die Zusammensetzung, nach dem Sprühen auf die Haut, auf der Haut gelassen wird oder in die Haut gerieben wird.

10. Zusammensetzung zur Verwendung in einem Verfahren nach irgendeinem vorhergehenden Anspruch, worin die Zusammensetzung eine nicht schäumende Lösung ist.

11. Zusammensetzung zur Verwendung in einem Verfahren nach irgendeinem vorhergehenden Anspruch, worin die Zusammensetzung ein anionisches Tensid umfasst.

12. Zusammensetzung zur Verwendung in einem Verfahren nach irgendeinem vorhergehenden Anspruch, welche 0,05 Gewichts-% Clobetasolpropionat, 49,25 Gewichts-% 92,8%iges Ethanol, 50,30 Gewichts% Isopropylmyristat und 0,1 Gewichts-% Natriumlaurylsulfat umfasst.

13. Zusammensetzung zur Verwendung in einem Verfahren nach irgendeinem vorhergehenden Anspruch, worin das Verfahren keine signifikante klinisch nachweisbare HPA-Achsensuppression verursacht.

## Revendications

1. Composition constituée de propionate de clobétasol, d'un composé de solvant et d'un émollient, la composition étant constituée de 0,05 % en poids de propionate de clobétasol, destinée à être utilisée dans un procédé de traitement d'un individu souffrant de lésions de psoriasis touchant au moins 2 % de la surface du corps de l'individu en vaporisant quotidiennement la composition sur les lésions de psoriasis pendant au moins 4 semaines.

2. Composition destinée à être utilisée dans un procédé selon la revendication 1, la composition étant dépourvue de pyrithione de zinc.

3. Composition destinée à être utilisée dans un procédé selon la revendication 1 ou 2, la composition étant vaporisée deux fois par jour pendant au moins quatre semaines.

4. Composition destinée à être utilisée dans un procédé selon l'une quelconque des revendications précédentes, dans laquelle le composé de solvant est de l'alcool éthylique.

5. Composition destinée à être utilisée dans un procédé selon l'une quelconque des revendications précédentes, dans laquelle l'émollient est du myristate d'isopropyle.

6. Composition destinée à être utilisée dans un procédé selon l'une quelconque des revendications précédentes, la composition étant constituée d'alcool, de myristate d'isopropyle et de laurylsulfate de sodium.

7. Composition destinée à être utilisée dans un procédé selon l'une quelconque des revendications précédentes, dans laquelle le support comprend un composant de solvant et un composant d'émollient dans un rapport compris entre 5:1 et 1:5 en poids.

8. Composition destinée à être utilisée dans un procédé selon la revendication 7, dans laquelle le rapport est de préférence compris entre 3:1 et 1:3.

9. Composition destinée à être utilisée dans un procédé selon l'une quelconque des revendications précédentes, la composition, après la vaporisation sur la peau, étant laissée sur la peau ou frottée sur la peau.

10. Composition destinée à être utilisée dans un procédé selon l'une quelconque des revendications précédentes, la composition étant une solution non moussante.

11. Composition destinée à être utilisée dans un procédé selon l'une quelconque des revendications précédentes, la composition comprenant un agent tensio-actif anionique.

12. Composition destinée à être utilisée dans un procédé selon l'une quelconque des revendications précédentes, constituée de 0,05 % en poids de propionate de clobétasol, de 49,25 % en poids d'éthanol à 92,8 %, de 50,30 % en poids de myristate d'isopropyle et de 0,1 % en poids de laurylsulfate de sodium.

13. Composition destinée à être utilisée dans un procédé selon l'une quelconque des revendications précédentes, le procédé ne provoquant aucune suppression significative cliniquement détectable de l'axe HHS.
